# Europäisches Patentamt

# European Patent Office

# Office européen des brevets

(19)

(11) Veröffentlichungsnummer: **0 143 857**
**B1**

(12) **EUROPÄISCHE PATENTSCHRIFT**

(45) Veröffentlichungstag der Patentschrift:
**06.04.88**

(21) Anmeldenummer: **83111993.8**

(22) Anmeldetag: **30.11.83**

(51) Int. Cl.⁴: **A 61 K 31/44**, A 61 K 9/48, A 61 K 47/00

(54) Coronartherapeutikum in Form von Weichgelatine-Kapseln.

(43) Veröffentlichungstag der Anmeldung:
**12.06.85 Patentblatt 85/24**

(45) Bekanntmachung des Hinweises auf die Patenterteilung:
**06.04.88 Patentblatt 88/14**

(84) Benannte Vertragsstaaten:
**AT BE CH DE FR GB IT LI LU NL SE**

(56) Entgegenhaltungen:
**DE-A-2 822 882**
**DE-C-1 000 135**
**LU-A-65 929**

**CHEMICAL ABSTRACTS, Band 99, Nr. 16, 17. Oktober 1983, Seite 360, Nr. 128364h, Columbus, Ohio, USA**

**Die Akte enthält technische Angaben, die nach dem Eingang der Anmeldung eingereicht wurden und die nicht in dieser Patentschrift enthalten sind.**

(73) Patentinhaber: **SIEGFRIED AKTIENGESELLSCHAFT, CH- 4800 Zofingen (CH)**

(72) Erfinder: **Dvorsky, Stephan, Hauptstrasse 80, CH- 4102 Binningen (CH)**
Erfinder: **Radivojevich, Franz, Dr., Ackerstrasse 5, CH- 4800 Zofingen (CH)**
Erfinder: **Joss, Hans, Hauptstrasse 292, CH- 4852 Murgenthal (CH)**

(74) Vertreter: **Fleck, Thomas, Dr.Dipl.- Chem., Patentanwälte Raffay, Fleck & Partner Postfach 32 32 17, D-2000 Hamburg 13 (DE)**

LIBER, STOCKHOLM 1988

## Beschreibung

Die vorliegende Erfindung betrifft ein Coronartherapeutikum in Form von Weichgelatine-Kapseln nach dem Oberbegriff des Anspruches 1.

Aus Chemical Abstracts, Band 99, Nummer 16 vom 17.10.83, Referat Nr. 128364 h, ist eine derartige Weichgelatine-Kapsel bekannt, die jedoch als Lösungsmittel Polyäthylenglykol und in Relation zum Gesamtgewicht maximal 1 Gew.-% Nifedipin aufweist.

Weitere Weichgelatine- oder auch Beißkapseln sind in der DE-PS 22 09d 526 beschrieben. Da Nifedipin bekannermaßen neben seiner starken Lichtempfindlichkeit auch noch schwer löslich ist, sind die gefärbten bzw. lichtdichten Kapseln unerwünscht groß, da eine relativ große Menge an Lösungsmitteln oder Lösungsvermittlern erforderlich ist. Dieser Nachteil wurde auch schon in der DE-OS 28 22 882 beschrieben, die deshalb eine Nifedipin enthaltende feste Präparat-Zusammensetzung mit kleineren Abmessungen vorschlägt. Die dortige Lehre ist jedoch auf die Herstellung von festen Arzneiformen gerichtet. Eine solche feste Präparatzusammensetzung hat den Nachteil, dass sie beim Schlucken von vielen Patienten als unangenehm empfunden wird und dass der Wirkstoff für den Organismus weniger schnell verfügbar wird als im Falle einer Weichgelatinekapsel, welche gewünschtenfalls vor dem Schlucken zerkaut werden kann, wobei der Wirkstoff schon im Mund perlingual teilweise resorbiert wird. Ebenfalls werden in der DE-OS 31 42 853 feste Arzneizubereitungen mit Nifedipin und Verfahren zu ihrer Herstellung beschrieben. Dort werden insbesondere die Nachteile bei der Verwendung des Nifedipin-PVP-Copräzipitats erwähnt, insbesondere mit Hinweis auf die sehr unterschiedlichen Wirkstoffgehalte in den einzelnen Tabletten oder Kapseln. Ein gleichmäßiger Wirkstoffgehalt soll nach der dortigen Lehre durch den Einsatz eines bestimmmten Gemisches von PVP mit Cellulose, Stärke und quervernetzten unlöslichen PVPP erzielt werden.

Aus dem weiteren Stand der Technik ist nach der DE-C-1,000,135 bekannt, THFP zur Herstellung stabiler Arzneilösungen zu verwenden, bei denen Acetylcholin und Pyridyl-3-carbiriol eingesetzt werden.

Der vorliegenden Erfindung liegt bei dem vorstehend genannten Stand der Technik die Aufgabe zugrunde, deren Nachteile zu vermeiden und insbesondere die eingangs genannte Weichgelatine-Kapsel derart zu verbessern, daß sie eine für die orale Applikation annehmbare Größe aufweist und gleichzeitig die Menge des Wirkstoffs Nifedipin beträchtlich erhöht und trotzdem gleichmäßig verteilt ist. Außerdem ist es ein Ziel der vorliegenden Erfindung, eine einfache Herstellung der Weichkapsel aus möglichst wenigen Einzelkomponenten zu gewährleisten.

Die vorstehende Aufgabe wird erfindungsgemäß durch eine Weichgelatine-Kapsel gelöst, aus einer Kapselhülle, die Glycerin, einen oder mehrere Farbstoffe mit einem Absorptionsbereich, der den zur Zersetzung des Wirkstoffes führenden Spektralbereich einschließt, sowie ein Opakisierungsmittel aufweist, und einer Kapselfüllung besteht, die den Wirkstoff Nifedipin (1,4-Dihydro-2,6-dimethyl-4-(2-nitrophenyl)-pyridin-3,5-dicarbonsäuredimethylester), sowie Polyvinylpyrrolidon (PVP), ein organisches Lösusngsmittel für Nifedipin und gegebenenfalls weitere Zusatzstoffe enthält, und die dadurch gekennzeichnet ist, daß die Kapselfüllung mindestens 4 Gewichtsprozent Nifedipin sowie Polyvinylpyrrolidon (PVP), beides gelöst in einem Gemisch von Polyätheralkoholen des Tetrahydrofurfurylalkohols (THFP), enthält, wobei Glycerin ausschließlich als Bestandteil in der Kapselhülle vorhanden ist.

Überraschenderweise hat sich also herausgestellt, daß man Nifedipin und Polyvinylpyrrolidon, das bei der Herstellung von Tabletten unerwünschte Wirkungen besitzt, hervorragend in Tetrahydrofurfuryl-alkoholpolyäthylen-glycoläther lösen kann, wodurch es als Flüssigkeit mit hohem und gleichmäßig verteiltem Wirkstoff an Nifedipin als flüssige Kapselfüllung für eine Weichgelatine-Kapsel geeignet ist. Als Ergebnis von Untersuchungen hat sich ferner gezeigt, daß bei der erfindungsgemäßen Kapselfüllung auf Glycerin als Zusatz verzichtet werden kann, ohne daß die optimale Bioverfügbarkeit oder die physikalischen Eigenschaften der Kapsel geschmälert werden. Darüber hinaus kann ebenfalls auf den Zusatz von Cellulose oder PVPP und dergleichen verzichtet werden, die nur unnötigerweise das Gewicht und den Umfang der Kapsel vergrößern würden.

Nach dem Stand der Technik werden zur Erzielung einer befriedigend stabilen und resorbierbaren Zubereitung Kapselfüllungen mit (bezogen auf das Wirkstoffgewicht) mindestens der 32-fachen Menge Lösungsmittel(n) und Hilfsstoffen benötigt, und der Kapselinhalt verkehrsüblicher Nifedipin-Beißkapseln weist in Übereinstimmung mit den in der DE-OS 22 09 526 offenbarten Beispiel einen Wirkstoffgehalt von bestenfalls 3 % auf. Nachdem die gewünschte, d.h. die derzeit als optimal geltende Nifedipinmenge pro Dosierungseinheit 5 bis 10 mg beträgt, bedingt dies bei der höheren der genannten Dosierungen eine Kapselfüllung, d.h. eine Gesamtmenge von Wirkstoff, Lösungsmitteln und Hilfsstoffen von über 380 mg mit einem Volumen von mindestens 0.36 ml. Daraus ergibt sich wiederum die Notwendigkeit zur Wahl einer sehr respektablen Kapselgröße von 6 Minims (1 Minim = ca. 0.06 ml; vgl. Martindale, The Extra Pharmacopoeia, London 1982, p.xxii) , was zu einem Gesamtgewicht der gefüllten Kapsel in Höhe von über 600 mg führt.

Bei erfindungsgemäßen Beißkapseln kann demgegenüber das Gewichtsverhältnis zwischen in der Kapselfüllung enthaltener Lösungsmittel- und Hilfsstoffmenge einerseits und Wirkstoffmenge andererseits so weit gesenkt werden, daß man bei 10 mg Wirkstoffgehalt mit einem Kapselvolumen von 2 Minims auskommt. Das Gewicht der gefüllten Kapsel kann demzufolge 300 mg wesentlich unterschreiten und vorzugsweise sogar auf weniger als 210 mg, beispielsweise bis auf 162 mg erniedrigt sein. Die demgemäß auch in ihren äußeren Abmessungen entscheidend verkleinerte Kapsel bedeutet für die der Therapie unterworfenen Patienten eine bedeutsame Erleichterung bei der oralen Einnahme.

2

Außerdem hat sich gezeigt, daß bisher bekannte Nifedipin-Zubereitungen bei in-vitro-Versuchen zum Auskristallisieren neigen, während der Inhalt der erfindungsgemäßen Kapsel fein amorph anfällt und so wesentlich besser resorbierbar ist.

Weitere Vorteile und Merkmale gehen aus den nachstehenden Unteransprüchen hervor, die ebenfalls von erfindungsgemäßer Bedeutung sein können. Insbesondere wird darauf verwiesen, daß schon ein äußerst geringer Zusatz von Polysorbat 80 die Benetzbarkeit verbessert.

Weiterhin wurde festgestellt, daß der erfindungsgemäße Kapselinhalt aufgrund seiner höheren Nifedipin-Konzentration sowohl licht- als auch temperaturbeständiger ist im Vergleich zu den bisher bekannten Weichgelatine-Kapseln, so daß die im Stand der Technik auftretenden Haltbarkeitsprobleme nunmehr glatt überwunden sind.

Die Herstellung solcher erfindungsgemäßer Weichkapsel wird insbesondere durchgeführt, indem man

1. Nifedipin, Polysorbat 80 und Polyvinylpyrrolidonin THFP (Tetrahydrofurfurylalkohol-polyäthylenglycoläther) löst und

2. die Lösung nach üblichen Verfahren in eine Weichgelatinekapselhülle eingebracht wird.

Andererseits kann man die Herstellung auch wie folgt durchführen.

1. Nifedipin, Polysorbat 80 und Polyvinylpyrrolidon wird in Ethanol gelöst. Das Lösungsmittel wird unter vermindertem Druck danach entfernt. Anschließend wird das Copräzipitat gemahlen.

2. Das Copräzipitat wird in THFP gelöst.

3. Die Lösung wird nach üblichen Verfahren in Weichgelatine-Kapseln, die entsprechend behandelt bzw. gefärbt sind, verkapselt.

Es dürfte einleuchten, daß neben Nifedipin und den erwähnten Hilfsstoffen selbstverständlich weitere übliche Formulierungshilfsmittel eingesetzt werden können, wobei trotzdem das Gesamtgewicht von 210 mg ± 5 % nicht überschritten wird.

Im folgenden werden zwei Beispiel für die erfindungsgemäße Weichgelatine-Kapsel angegeben:

<u>Nifedipin Weichgelatine-Kapseln 5 mg</u>
Größe: 2 Minims oval (0,12 ml)
Zusammensetzung
1 Weichgelatine-Kapsel enthält
Kapselinhalt

| | | |
|---|---|---|
| Nifedipin | 5,00 mg | = 4,46 % |
| Polyvinylpyrrolidon | 14,98 mg | |
| Polysorbat 80 | 0,02 mg | |
| THFP* | 92,00 mg | |

*Tetrahydrofurfurylalkohol-polyäthylenglycoläther (Tetraglycol)

| | |
|---|---|
| Kapselhülle | |
| Gelatine | 35,42 mg |
| Glycerin 85 % | 8,05 mg |
| Sorbit | 5,90 mg |
| Titandioxid, E 171 | 0,18 mg |
| Eisenoxidschwarz, E 172 g | 0,07 mg |
| Cochenilerot A 62,5 %, E 124 | 0,38 mg |
| Konservierungsmittel | 0,35 mg |
| | ———— |
| | 162,35 mg |

<u>Nifedipin Weichgelatine-Kapseln 10 mg</u>
Größe: 2 Minims oval (0,12 ml)
Zusammensetzung
1 weichgelatine-Kapsel enthält
Kapselinhalt

| | | |
|---|---|---|
| Nifedipin | 10,00 mg | = 8,93 % |
| THFP* | 72,00 mg | |
| Polyvinylpyrrolidon | 29,96 mg | |
| Polysorbat 80 | 0,04 mg | |

| Kapselhülle | |
|---|---|
| Gelatine | 35,42 mg |
| Glycerin 85 % | 8,05 mg |
| Sorbit | 5,90 mg |
| Titandioxid, E 171 | 0,18 mg |
| Eisenoxidschwarz, E 172 | 0,07 mg |
| Cochenilerot A 62,5 %, E 124 | 0,38 mg |
| Konservierungsmittel | 0,35 mg |
| | ———————— |
| | 162,35 mg |

˙Tetrahydroforfurylalkohol-polyäthylenglycoläther (Tetraglycol)

Die erfindungsgemäße Weichgelatine-Kapsel gemäß dem Beispiel 1 und dem Beispiel 2 wiegen also insgesamt nur 162 mg und sind somit nur etwa 1/4 so groß wie die vorbekannten Kapseln. Gleichzeitig zeigen die Gewichtsprozentangaben für Nifedipin mit 4,46 und 8,93 % bezogen auf den Kapselinhalt eine deutlich höhere Konzentration als der Stand der Technik bei flüssigen Füllungen für solche Weichkapseln.

Ferner kann bei der Kapselhülle beispielsweise auch auf Sorbit verzichtet werden, die dann etwa folgende Zusammensetzung aufweist:

| Gelatine | 45,900 mg |
|---|---|
| Glycerin | 37,800 mg |
| | |
| TiO$_2$ | 0,324 mg |
| Eisenoxid-schwarz | 0,126 mg |
| Ponceau red E 124 | 0,684 mg |
| Wassergehalt ca. | 5,166 mg |

**Patentansprüche**

1. Coronartherapeuticum in Form von Weichgelatine-Kapseln, bestehend aus einer Kapselhüllle, die Glycerin, einen oder mehrere Farbstoffe mit einem Absorptionsbereich, der den zur Zersetzung des Wirkstoffes führenden Spektralberich enschließt, sowie ein Opakisierungsmittel aufweist, und einer Kapselfüllung, die den Wirkstoff Nifedipin (1-4-Dihydro-2,6-dimethyl-4-(2-nitrophenyl)-pyridin-3,5-dicarbonsäuredimethylester), sowie Polyvinylpyrrolidon (PVP), ein organisches Lösungsmittel für Nifedipin und gegebenenfalls weitere Zusatzstoffe enthält, dadurch gekennzeichnet, daß die Kapselfüllung mindestens 4 Gewichtsprozent Nifedipin sowie Polyvinylpyrrolidon (PVP), beides gelöst in einem Gemisch von Polyätheralkoholen des Tetrahydrofurfurylalkohols (THFP), enthält, wobei Glycerin ausschließlich als Bestandteil in der Kapselhülle vorhanden ist.

2. Coronartherapeutikum nach Anspruch 1, gekennzeichnet durch ein Gewicht der gefüllten Kapsel von höchstens 300 mg und vorzugsweise höchstens 210 mg.

3. Coronartherapeutikum nach Anspruch 1 oder 2, dadurch gekennzeichnet, daß das PVP ein Molekulargewicht von etwa 25,000 bis 40,000 aufweist.

4. Coronartherapeutikum nach Anspruch 1, dadurch gekennzeichnet, daß die Kapselfüllung außerdem eine geringe Menge Polysorbat 80 als Tensid enthält.

5. Coronartherapeutikum nach Anspruch 1, dadurch gekennzeichnet, daß als Farbstoff ein Gemisch von Eisenoxidschwarz oder -rot (E 172) und/oder Cochenilerot A 62,5 %. (E 124) und/oder Cochineal (E 120) und/oder Erythrosine (E 127) in der Kapselhülle enthalten ist.

6. Coronartherapeutkum nach Anspruch 1, dadurch gekennzeichnet, daß als Opakisierungsmittel Titandioxid in der Kapselhülle enthalten ist.

7. Coronartherapeutikum nach Anspruch 1, dadurch gekennzeichnet, daß die Kapselhülle neben Farbstoff, dem Opakisierungsmittel und Glycerin Sorbit enthält.

8. Verfahren zur Herstellung des Coronartherapeutikums nach Anspruch 1 oder einem oder mehreren der Ansprüche 2 bis 7, dadurch gekennzeichnet, daß für die Zubereitung der Kapselfüllung Nifedipin, PVP und gegebenenfalls Polysorbat in THFP gelöst werden.

9. Verfahren nach Anspruch 8, dadurch gekennzeichnet, daß das Nifedipin, das PVP und gegebenenfalls das Polysorbat in Ethanol gelöst und dann durch Verdampfen des Lösungsmittels Kopräzipitiert werden und daß anschließend das Kopräzipitat in THFP gelöst wird.

## Claims

1. Coronary therapeutic in the form of soft gelatine capsules consisting of a capsule casing which contains glycerol, one or several colourants with an absorption region which includes the spectral region which gives rise to the decomposition of the filler, as well as an opacifying agent, and a capsule filling which contains the active substance Nifedipin (1,4-dihydro-2,6-dimethyl-4-(2-nitrophenyl)-pyridine-3,5-dicarboxylic acid dimethyl ester) as well as polyvinylpyrrolidone (PVP), an organic solvent for Nifedipin and optionally further additives, characterised in that the capsule filling contains at least 4 % by weight of Nifedipin as well as polyvinylpyrrolidone (PVP), both dissolved in a mixture of polyether alcohols of tetrahydrofurfuryl alcohols (THFP), glycerol being present exclusively as constituent in the capsule casing.

2. Coronary therapeutic according to claim 1, characterised by a weight of the filled capsule of at the most 300 mg and preferably at the most 210 mg.

3. Coronary therapeutic according to claim 1 or 2, characterised in that the PVP possesses a molecular weight of about 25,000 to 40,000.

4. Coronary therapeutic according to claim 1, characterised in that the capsule filling contains in addition a small amount of Polysorbate 80 as surfactant.

5. Coronary therapeutic according to claim 1, characterised in that there is contained in the capsule casing as colourant a mixture of black iron oxide or red iron oxide (E 172) and/or Cochineal red A 62,5% (E 124) and/or Cochineal (E 120) and/or Erythrosine (E 127).

6. Coronary therapeutic according to claim 1, characterised in that titanium dioxide is contained in the capsule casing as opacifying agent.

7. Coronary therapeutic according to claim 1, characterised in that the capsule casing contains sorbitol in addition to colourant, the opacifying agent and glycerol.

8. Process for the production of the coronary therapeutic according to claim 1 or one or several of claims 2 to 7, characterised in that, in the preparation of the Nifedipin capsule filling, PVP and possibly polysorbate are dissolved in THFP.

9. Process according to claim 8, characterised in that the PVP and optionally the polysorbate are dissolved in ethanol and then are coprecipitated by evaporation of the solvent and that subsequently the coprecipitate is dissolved in THFP.

## Revendications

1. Médicament pour le traitement des affections des coronaires, sous forme de capsules en gélatine molle, consistant en une enveloppe de capsule qui comporte du glycérol, un ou plusieurs colorants ayant un domaine d'absorption comprenant le domaine spectral provoquant la décomposition de la substance active, ainsi qu'un opacifiant, et un garnissage de capsule contenant la substance active nifédipine (dihydro-1,4 diméthyl-2,6 (nitro-2 phényl)-4 pyridine dicarboxylate-3,5 de diméthyle) ainsi que de la polyvinylpyrrolidone (PVP), un solvant organique de la nifédipine et éventuellement d'autres additifs, médicament caractérisé en ce que le garnissage de la capsule contient au moins 4 % en poids de nifédipine ainsi que de la polyvinylpyrrolidone (PVP), toutes deux dissoutes dans un mélange de polyétheralcools de l'alcool tétrahydrofurfurylique (THFP), le glycérol étant exclusivement présent comme constituant de l'enveloppe de capsule.

2. Médicament pour le traitement des affections des coronaires selon la revendication 1, caractérisé en ce que le poids de la capsule emplie est au maximum de 300 mg et, avantageusement, au maximum de 210 mg.

3. Médicament pour le traitement des affections des coronaires selon la revendication 1 ou 2, caractérisé en ce que la PVP (polyvinylpyrrolidone) présente un poids moléculaire compris entre environ 25 000 et 40 000.

4. Médicament pour le traitement des affections des coronaires selon la revendication 1, caractérisé en ce que le garnissage de capsule contient en outre une faible quantité de "Polysorbat 80" comme tensio-actif.

5. Médicament pour le traitement des affections des coronaires selon la revendication 1, caractérisé en ce qu'un mélange de noir ou rouge d'oxyde de fer (E 172) et/ou rouge de cochenille à 62,5 % (E 124) et/ou du cochinéal (E 120) et/ou de l'érythrosine (E 127) est contenu comme colorant dans l'enveloppe de capsule.

6. Médicament pour le traitement des affections des coronaires selon la revendication 1, caractérisé en ce que du bioxyde de titane est contenu comme opacifiant dans l'enveloppe de capsule.

7. Médicament pour le traitement des affections des coronaires selon la revendication 1, caractérisé en ce que l'enveloppe de capsule contient du sorbitol en plus du colorant, de l'opacifiant et du glycérol.

8. Procédé pour préparer le médicament pour le traitement des affections des coronaires selon la revendication 1 ou selon une ou plusieurs des revendications 2 à 7, caractérisé en ce que, pour préparer le garnissage de capsules, on dissout dans THFP la nifédipine, la polyvinylpyrrolidone et éventuellement du "Polysorbat".

9. Procédé selon la revendication 8, caractérisé en ce qu'on dissout la nifédipine, la polyvinylpyrrolidone et éventuellement le "Polysorbat" dans de l'éthanol et on les coprécipite ensuite par évaporation du solvant, et l'on dissout ensuite le coprécipité dans THFP.